# EUROPÄISCHE PATENTSCHRIFT

(11) **EP 1 801 099 B1**
(45) Veröffentlichungstag und Bekanntmachung des Hinweises auf die Patenterteilung: **09.03.2011**
(21) Anmeldenummer: 07104119.8
(22) Anmeldetag: 10.09.2004
(51) Int. Cl.: C07C 323/19, C07D 301/12

(54) **Dithiolmischungen zum Entfernen von Hornsubstanzen aus Häuten**
Dithiol mixtures in order to remove horny substances of skins
Mixtures dithioles pour enloigner des composés cornés des peaux

(30) Priorität: 17.09.2003 DE 10343252
(43) Veröffentlichungstag der Anmeldung: 27.06.2007
(62) Teilanmeldung aus: 04765052.8
(73) Patentinhaber: BASF SE, 67056 Ludwigshafen (DE)
(72) Erfinder: Bühler, Holger, 68163, Mannheim (DE); Teles, Joaquim Henrique, 67166, Otterstadt (DE); Pabst, Gunther, 92318, Neumarkt (DE); Taeger, Tilman Lüdecke, 64342, Seeheim-Jugenheim (DE)

(56) Entgegenhaltungen:
- DE-A1- 2 131 630

## Beschreibung

### Erfindungsgemäß

Die vorliegende Erfindung betrifft Dithiolgemische, enthaltend im Bereich von
(A) 55 bis 65 mol-%, bevorzugt 59 bis 61 mol-% erythro-Dithiol und
(B) 35 bis 45 mol-%, bevorzugt 39 bis 41 mol-% threo-Dithiol der allgemeinen Formel IV 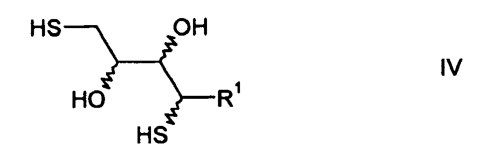 und korrespondierende Salze von erfindungsgemäßen Dithiolgemischen, wobei

- R¹: gewählt wird aus Wasserstoff und C₁-C₁₂-Alkyl, unsubstituiert oder substituiert mit einer oder mehreren S-H oder O-H-Gruppen.

Dithiole sind äußerst vielseitige Reagenzien. So ist bekannt, dass man 1,4-Dimercaptobutandiol bei der Aufreinigung und Stabilisierung von Enzymen (Schutzreagens für-SH Gruppen, W.W. Cleland, Biochemistry, 3, 480, 1964) einsetzen kann. Weiterhin ist aus DE 22 09 458 bekannt, dass sogenanntes 1,4-Dithiol-2,3-butandiol und seine Metallsalze als Haarwellmittel und Enthaarungsmittel verwendet werden können. Aus DE 23 37 101 ist bekannt, dass man n-Butan-2,3-diol-1,4-dithiol und seine Alkalimetallsalze zur Erzeugung von Dauerwellen verwenden kann. Aus DE 21 31 630 ist bekannt, dass man Mittel, bestehend aus mindestens 0,25 Gew.-% Dimercaptobutandiol und etwa 0,01 bis 40 Gew.-% einer wasserlöslichen Guanidinverbindung und einem pH-Wert von unter 12 auf Meerschweinchen aufbringen kann, um sie zu enthaaren, oder auf menschliche Hornhaut, um Schwielen zu beseitigen, ohne dass es zu Hautreizungen bei Meerschweinchen oder gar zu Erythrämie (bösartige Wucherungen des Bildungssystems der roten Blutkörperchen) kommt. Die Epidermis bleibt bei der in DE 21 31 630 beschriebenen Behandlung erhalten.

Es ist also wünschenswert, eine Syntheseroute zu finden, durch die man n-Butan-2,3-diol-1,4-dithiol und Derivate mit guter Ausbeute und in guter Reinheit gewinnen kann.

Aus DE 22 09 458 und DE 23 37 101 ist bekannt, dass man racemisches Dithiolbutandiol, verunreinigt mit geringfügigen Mengen an Dithioerythrol, herstellen kann, indem man Butadienbisepoxid mit Schwefelwasserstoff in Gegenwart eines alkalischen Katalysators im Bereich zwischen 15 und 40°C in einem Schwefelwasserstoff lösenden Mittel umsetzt, dessen Volumen zum Volumen an Butadienbisepoxid im Verhältnis von mindestens 5:1 steht, und das so erhältliche Dithiolbutandiol abtrennt.

Aus US 5,329,024 ist bekannt, dass man Olefine in Gegenwart von Mangan-Komplexen mit Hilfe von großen molaren Überschüssen an H₂O₂, beispielsweise Olefin : H₂O₂ wie 1:100) zu Epoxiden umsetzen kann.

Aus D. de Vos et al., Tetrahedron Lett. 1998, 39, 3221 ist bekannt, dass man Bisepoxide von Isopren und von 4-Vinyl-cyclohexen herstellen kann, indem man die betreffenden Diene mit einem großen Überschuss (Molverhältnis etwa 12:1) H₂O₂ in Gegenwart eines Mangan-Komplexes umsetzt. Trotz der großen Überschüsse an H₂O₂ fallen jedoch beträchtliche Anteile an Monoepoxiden an. Auch ist die Ausbeute an gewünschten Bisepoxiden noch verbesserungsfähig.

Es bestand also die Aufgabe, neue Gemische von Dithiolen und Verwendungen für Gemische von Dithiolen bereit zustellen.

Demgemäß wurde die eingangs definierten Ditiolgemische gefunden.

Erfindungsgemäße Dithiolgemische enthalten Verbindungen, die in Fischer-Projektion wie folgt abgebildet werden können:

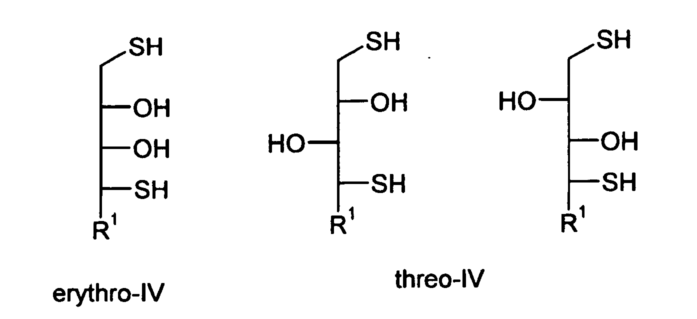

Erfindungsgemäße Dithiolgemische können korrespondierende Salze von erythro-IV und threo-IV enthalten.

In einer Ausführungsform der vorliegenden Erfindung liegt threo-IV als Racemat vor.
- R¹ wird ausgewählt aus: C₁-C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl oder n-Decyl, besonders bevorzugt C₁-C₄-Alkyl wie Methyl, Ethyl, n-Propyl, iso-Propyl, n-Butyl, iso-Butyl, sec.-Butyl und tert.-Butyl;
C₁-C₁₂-Alkyl, substituiert mit einer oder mehreren Hydroxy- oder Thiolgruppen wie Hydroxymethyl, 2-Hydroxyethyl, 1,2-Dihydroxyethyl, 3-Hydroxy-n-propyl, 2-Hydroxy-iso-Propyl, ω-Hydroxy-n-butyl, ω-Hydroxy-n-decyl, HS-CH₂-;
HS-(CH₂)₂- oder HS-(CH₂)₃-,
und ganz besonders bevorzugt Wasserstoff.

In einer Ausführungsform der vorliegenden Erfindung, in der R¹ gewählt wird aus unsubstituiertem oder substituiertem C₁-C₁₂-Alkyl, liegt erythro-IV als Racemat vor.

Erfindungsgemäße Dithiolgemische können mit geringen Anteilen an Hydroxythiol der allgemeinen Formeln VI.1 oder VI.2 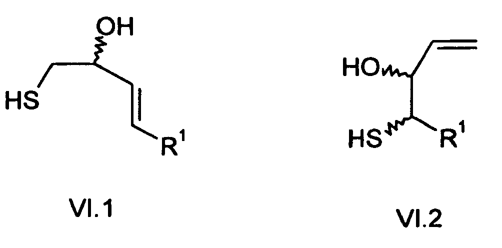 verunreinigt sein, wobei der Anteil an Hydroxythiol im allgemeinen 8 mol-%, bezogen auf reines Dithiol bzw. korrespondierendes Salz von Dithiol der allgemeinen Formel IV, nicht übersteigt.

Unter den korrespondierenden Salzen sind insbesondere die Mono- und Dinatriumsalze, Mono- und Dikaliumsalze sowie Kaliumnatriumsalze von Dithiolen der allgemeinen Formel IV zu nennen, weiterhin die entsprechenden Calcium- und Magnesiumsalze. Auch sind die Ammoniumsalze bzw. primären, sekundären, tertiären und insbesondere quartären Mono- und Diammoniumsalze zu nennen.

Bevorzugte Mono- und Diammoniumsalze haben als Kationen solche der Formel N(R³)(R⁴)(R⁵)(R⁶)⁺, wobei R³ bis R⁶ jeweils gleich oder verschieden sind und ausgewählt aus Wasserstoff, C₁-C₁₂-Alkyl, Phenyl oder CH₂-CH₂-OH. Beispielhaft seien Tetramethylammonium, Tetraethylammonium, Methyldiethanolammonium und n-Butyldiethanolammonium genannt.

Ein weiterer Gegenstand der vorliegenden Erfindung sind wässrige Lösungen, enthaltend erfindungsgemäßes Dithiolgemisch. Erfindungsgemäße wässrige Lösungen kann man beispielsweise dadurch erhalten, dass man erfindungsgemäßes Dithiolgemisch oder korrespondierendes Salz in Wasser löst. Vorzugsweise haben erfindungsgemäße wässrige Lösungen einen Feststoffgehalt von 0,1 bis 50 Gew.-%.

Es wurde nun gefunden, dass erfindungsgemäße Dithiolgemische und ihre korrespondierenden Salze, beispielsweise in Form ihrer wässrigen Lösung, vorzüglich zur Behandlung von Häuten toter Tiere eingesetzt werden können.

Gegenstand der vorliegenden Erfindung ist daher die Verwendung von erfindungsgemäßen Dithiolgemischen zum Behandeln von Häuten toter Tiere.

Gegenstand der vorliegenden Erfindung ist ferner ein Verfahren zum Entfernen von Hornsubstanzen aus Häuten toter Tiere unter Verwendung von mindestens einem erfindungsgemäßen Dithiolgemisch, im Folgenden auch erfindungsgemäßes Behandlungsverfahren genannt.

Unter Hornsubstanzen werden im Sinne der vorliegenden Erfindung Schwielen, Federn, Nägel- und Krallenteile und insbesondere Haare von Tieren verstanden.

Häute toter Tiere können noch Reste von Fleisch der betreffenden toten Tiere enthalten. Erfindungswesentlich ist jedoch, dass sie Hornsubstanzen enthalten. Dabei ist die Menge an Hornsubstanz, bezogen auf das Gesamtgewicht der Haut bzw. des Pelzes oder des Pelzfells, unkritisch. Das erfindungsgemäße Verfahren eignet sich sowohl zur Entfernung von großen Mengen an Hornsubstanz als auch beispielsweise zur Entfernung kleiner Haarreste.

Unter toten Tieren werden im Sinne der vorliegenden Erfindung nicht nur geschlachtete oder auf andere Art getötete Tiere verstanden, sondern auch solche Tiere, die aufgrund von Unfällen, beispielsweise Verkehrsunfällen oder Kämpfen mit Artgenossen oder anderen Tieren, oder durch natürliche Ursachen wie Alter oder Krankheit verendet sind.

Bei den Häuten toter Tiere handelt es sich üblicherweise um Häute von Rindern, Kälbern, Schweinen, Ziegen, Schafen, Lämmern, Elchen, Wild wie beispielsweise Hirschen oder Rehen, weiterhin Vögeln wie beispielsweise Straußen, Fischen oder Reptilien wie beispielsweise Schlangen.

Zur Ausführung des erfindungsgemäßen Behandlungsverfahrens geht man vorteilhaft wie folgt vor.

Man versetzt mindestens eine Haut oder Hautteile von mindestens einem toten Tier mit mindestens einem erfindungsgemäßen Dithiolgemisch.

Im Allgemeinen genügt eine Menge von 0,1 bis 5 Gew.-% mindestens eines erfindungsgemäßen Dithiolgemischs, bezogen auf das Haut- bzw. Salzgewicht der Haut,. Bevorzugt sind 0,5 bis 2,5 Gew.-%, besonders bevorzugt sind 0,75 bis 1,5 Gew.-%.

Bevorzugt erfolgt die erfindungsgemäße Behandlung der Häute mit mindestens einem erfindungsgemäßen Dithiolgemisch im Äscher bzw. der Schwöde, und zwar unter haarzerstörenden oder auch unter haarerhaltenden Bedingungen. Dabei gelingt es, im Äscher bzw. der Schwöde statt der üblichen Konzentration von etwa 4 Gew.-% Na₂S bzw. NaHS oder sogar geringfügig mehr, mit einer Konzentration von weniger als 1 Gew.-% Na₂S bzw. NaHS bei gleich großer oder gar besserer Wirkung bezüglich der Entfernung von Hornsubstanzen auszukommen.

In einer Variante des erfindungsgemäßen Verfahrens setzt man im Äscher mindestens ein erfindungsgemäßes Dithiolgemisch zusammen mit aus der Gerberei bekannten Thiolen wie beispielsweise Mercaptoethanol oder Thioglykolsäure ein. Bevorzugt setzt man weniger als 0,5 Gew.-% Mercaptoethanol oder Thioglykolsäure ein.

In einer ganz besonders bevorzugten Variante des erfindungsgemäßen Verfahrens gelingt es jedoch, auf den Einsatz von Na₂S bzw. NaHS oder anderer übel riechender schwefelhaltiger Reagenzien zu verzichten.

In einer Ausführungsform der vorliegenden Erfindung behandelt man Häute in einer wässrigen Flotte. Dabei kann das Flottenverhältnis von 1:10 bis 10:1, bevorzugt 1:2 bis 4:1, besonders bevorzugt bis 3:1 betragen, bezogen auf das Hautgewicht bzw. Salzgewicht der Häute.

In einer Ausführungsform der vorliegenden Erfindung kann man das erfindungsgemäße Behandlungsverfahren bei pH-Werten von 7 bis 14, bevorzugt von 8 bis 13 und besonders bevorzugt von 9 bis 12,5 durchführen.

Zur Einstellung des pH-Werts kann man so vorgehen, dass man bis zu 3 Gew.-% Kalk (auch Kalkhydrat), bezogen auf die Flotte, zugibt. Man kann aber auch die Kalkmenge deutlich reduzieren. In einer bevorzugten Variante des erfindungsgemäßen Behandlungsverfahrens verzichtet man auf den Einsatz von Kalk. In der bevorzugten Ausführungsform setzt man eine oder mehrere anorganische basische Alkalimetallverbindungen zu, beispielsweise ein oder mehrere Hydroxide oder Carbonate von Alkalimetallen, bevorzugt von Natrium oder Kalium und ganz besonders bevorzugt von Natrium. Andere geeignete anorganische basische Alkalimetallverbindungen sind Alkalimetallsilikate. Man kann auch basische Amine, beispielsweise Ammoniak, Methylamin, Dimethylamin, Ethylamin oder Triethylamin zusetzen oder Kombinationen aus Alkalimetallverbindung und einem oder mehreren basischen Aminen.

Neben Wasser können noch weitere organische Lösemittel in der Flotte sein, beispielsweise bis zu 20 Vol.-% Ethanol oder Isopropanol.

Das erfindungsgemäße Behandlungsverfahren lässt sich in gerbereiüblichen Gefäßen durchführen, in denen üblicherweise geäschert wird. Vorzugsweise führt man das erfindungsgemäße Behandlungsverfahren in drehbaren Fässern mit Einbauten durch. Die Drehzahl beträgt üblicherweise 0,5 bis 100/min, bevorzugt 1,5 bis 10/min und besonders bevorzugt bis 4,5/min.

Die Druck- und Temperaturbedingungen zur Durchführung des erfindungsgemäßen Behandlungsverfahrens sind im Allgemeinen unkritisch. Als geeignet hat sich die Durchführung bei Atmosphärendruck erwiesen; ein auf bis zu 10 bar erhöhter Druck ist ebenfalls denkbar. Geeignete Temperaturen sind 10 bis 45°C, bevorzugt 15 bis 35°C und besonders bevorzugt 25 bis 30°C.

Man kann mindestens ein erfindungsgemäßes Dithiolgemisch am Beginn des erfindungsgemäßen Behandlungsverfahrens dosieren, man kann aber zunächst auch die Häute unter basischen Bedingungen einweichen und erst nach einiger Zeit mindestens ein erfindungsgemäßes Dithiolgemisch dosieren. Die Dosierung kann in einem Schritt erfolgen, d.h. die Gesamtmenge an erfindungsgemäßem Dithiolgemisch wird in einem Schritt dosiert; man kann aber erfindungsgemäßes Dithiolgemisch auch portionsweise oder kontinuierlich dosieren.

Das erfindungsgemäße Behandlungsverfahren lässt sich in einem Zeitraum von 10 Minuten bis 48 Stunden, bevorzugt 1 bis 36 Stunden und besonders bevorzugt 3 bis 15 Stunden durchführen.

Selbstverständlich kann man zur Ausübung des erfindungsgemäßen Behandlungsverfahrens noch gerbereiübliche Hilfsstoffe zusetzen, beispielsweise Phosphine, wie z. B. Triphenylphosphin oder Tris(2-Carboxyethyl)-phosphinhydrochlorid, weiterhin Hydroxylamin, Harnstoff, Guanidin bzw. Guanidinium-Hydrochlorid, Hydrazin, Biozide, Enzyme, Tenside und Emulgatoren.

Durch das erfindungsgemäße Behandlungsverfahren lassen sich vorzüglich enthaarte Blößen herstellen. Überraschend findet man, dass auch die Epidermis bereits nach kurzer Behandlungsdauer vollständig oder doch zumindest weitgehend abgelöst wird.

Weiterhin wurde gefunden, dass sich nach dem erfindungsgemäßen Behandlungsverfahren hergestellte Blößen ganz vorzüglich zur Herstellung von Leder eignen. Nach gerbereiüblicher Weiterverarbeitung von nach dem erfindungsgemäßen Behandlungsverfahren hergestellten Blößen, d.h. Beizen, ggf. Entkälken, Pickeln, chromfreies Gerben oder Chromgerbung, Nachgerben und Zurichten beobachtet man, dass man nach dem erfindungsgemäßen Behandlungsverfahren hergestellte Blößen zu Leder mit einer verbesserten Flächenausbeute und geringeren Schwellungsschäden weiterverarbeiten kann, verglichen mit Leder, das aus Blößen hergestellt wird, die mit Hilfe von beispielsweise Na₂S, NaHS, Thioglykolsäure oder Aminethanol enthaart wurden.

Die Herstellung von erfindungsgemäßen Dithiolgemischen kann man beispielsweise so durchführen, dass man
(a) ein konjugiertes Dien der allgemeinen Formel I 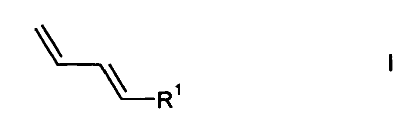 in dem
   - R¹: gewählt wird aus Wasserstoff und C₁-C₁₂-Alkyl, unsubstituiert oder substituiert mit einer oder mehreren S-H oder O-H-Gruppen, in Gegenwart eines Katalysators, der erhältlich ist durch Kontaktieren von mindestens einer Manganverbindung, gewählt aus A₂MnX₄, AMnX₃, MnY, MnX₂ und MnX₃ mit mindestens einem Liganden L der allgemeinen Formel II 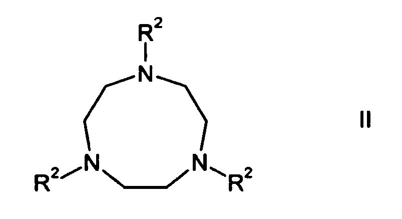
   wobei die Variablen wie folgt definiert sind:
   - X: verschieden oder gleich und gewählt aus einwertigen Anionen,
   - Y: ein zweiwertiges Anion,
   - A: gewählt aus Alkalimetall und Ammonium, welches alkyliert sein kann,
   - R²: verschieden sind oder vorzugsweise gleich und gewählt aus C₁-C₂₀-Alkyl, und mindestens einem Coliganden, der von Monocarbonsäuren, zwei- oder mehrwer- tigen Carbonsäuren oder Diaminen abgeleitet ist,
   mit mindestens einem Peroxid umsetzt, wobei man bis zu 4 Äquivalenten Peroxid pro Äquivalent C-C-Doppelbindung einsetzt, und
(b) ohne Isolierung von in Stufe (a) hergestelltem Bisepoxid in Gegenwart mindestens eines basischen Katalysators mit H₂S umsetzt.

Dazu setzt man ein konjugiertes Dien der allgemeinen Formel I um, 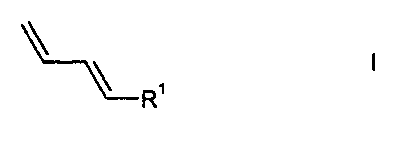 in dem die Variable R¹ wie vorstehend definiert ist.

Natürlich kann man auch Mischungen von Olefinen oder Dienen umsetzen, die konjugiertes Dien der allgemeinen Formel I enthalten.

Die Umsetzung erfolgt in Gegenwart von Katalysator, der erhältlich ist
durch Kontaktieren von mindestens einer Manganverbindung, gewählt aus A₂MnX₄, AMnX₃, MnY, MnX₂ und MnX₃
mit mindestens einem Liganden L der allgemeinen Formel II 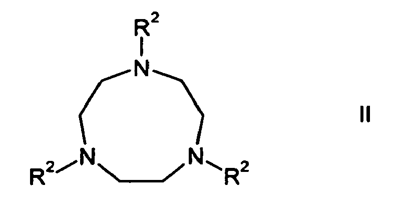 und mindestens einem Coliganden, der von Monocarbonsäuren, zwei- oder mehrwertigen Carbonsäuren oder Diaminen abgeleitet ist,
wobei die Variablen wie folgt definiert sind:
- X: verschieden oder gleich und gewählt aus einwertigen Anionen, R³O⁻, F⁻, Cl⁻, Br⁻, I⁻ , NCS⁻, N₃⁻, I₃⁻, R³COO⁻, R³SO₃⁻, R³SO₄⁻, OH⁻, CN⁻, OCN⁻, NO₃⁻, ClO₄⁻, PF₆⁻, BPh₄ mit Ph = Phenyl und F₃CSO₃⁻. Besonders bevorzugt sind Cl⁻ und Acetat.
- Y: ein zweiwertiges Anion, besonders bevorzugt SO₄²⁻ und HPO₄²⁻.
- A: verschieden oder vorzugsweise gleich und gewählt aus Alkalimetallkationen, bei- spielsweise Li⁺, Na⁺, K⁺, Rb⁺ und Cs⁺, insbesondere Na⁺ und K⁺ und Ammonium NH₄⁺, welches alkyliert sein kann, beispielsweise N(R⁴)(R⁵)(R⁶)(R⁷)⁺, wobei R⁴ bis R⁷ jeweils gleich oder verschieden sind und ausgewählt aus Wasserstoff, Benzyl, C₁-C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.- Pentyl, neo-Pentyl, 1,2-Dimethylpropyl, iso-Amyl, n-Hexyl, iso-Hexyl, sec.-Hexyl oder n-Decyl, besonders bevorzugt C₁-C₄-Alkyl wie Methyl, Ethyl, n-Propyl, iso- Propyl, n-Butyl, iso-Butyl, sec.-Butyl und tert.-Butyl, Phenyl oder CH₂-CH₂-OH. Beispielhaft seien Tetramethylammonium, Tetraethylammonium, Methyldiethano- lammonium und n-Butyldiethanolammonium genannt.
- R²: verschieden oder vorzugsweise gleich und gewählt aus verzweigtem oder vor- zugsweise unverzweigtern C₁-C₂₀-Alkyl wie beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.- Hexyl, n-Octyl, n-Decyl, n- Dodecyl, n-Tetradecyl, n-Hexadecyl, n-Octadecyl und n-Eicosyl, bevorzugt unverzweigtes C₁-C₁₂-Alkyl wie Methyl, Ethyl, n-Propyl, n-Butyl, n-Pentyl, n-Hexyl, n-Heptyl, n-Octyl, n-Decyl oder n-Dodecyl, besonders bevorzugt C₁-C₄-Alkyl wie Methyl, Ethyl, n-Propyl, n-Butyl, und ganz besonders bevorzugt Methyl.
- R³: steht vorzugsweise für
C₁-C₂₀-Alkyl, beispielsweise Methyl, Ethyl, n-Propyl, iso-Propyl, n-Butyl, iso-Butyl, sec.- Butyl, tert.-Butyl, n-Pentyl, iso-Pentyl, sec.-Pentyl, neo-Pentyl, 1,2-Dimethyl- propyl, iso-Amyl, n-Hexyl, iso-Hexyl, sec.-Hexyl, n-Octyl, n-Decyl, n- Dodecyl, n-Tetradecyl, n-Hexadecyl, n-Octadecyl und n-Eicosyl, bevorzugt unverzweigtes C₁-C₁₂-Alkyl wie Methyl, Ethyl, n-Propyl, n-Butyl, n-Pentyl, n-Hexyl, n-Heptyl, n-Octyl, n-Decyl oder n-Dodecyl, besonders bevorzugt C₁-C₄-Alkyl wie Methyl, Ethyl, n-Propyl, n-Butyl, und ganz besonders bevorzugt Methyl,
substituiertes C₁-C₂₀-Alkyl wie beispielsweise ω-Cyclohexylpropyl, 2-Cyclohexylethyl;
C₃-C₁₂-Cycloalkyl wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl,
C₆-C₁₄-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevor- zugt Phenyl
oder Benzyl.

Besonders bevorzugte Beispiele für eingesetzte Manganverbindungen sind Mangan(II)sulfat, Mangan(II)acetat, Mangan(II)chlorid, Mangan(II)perchlorat oder Kaliumhexachloromanganat(IV) K₂MnCl₆.

Dabei ist es möglich, dass eingesetzte Manganverbindungen Kristallwasser und/oder Hydratwasser aufweisen wie beispielsweise Mn(OAc)₂·4 H₂O, MnSO₄·H₂O, Mn(ClO₄)₂·6 H₂O, MnCl₂·4 H₂O.

In einer Ausführungsform der Herstellung setzt man im Bereich von 0,001 bis 0,1, besonders bevorzugt 0,005 bis 0,01 Äquivalente Manganverbindung ein, bezogen auf Dien der allgemeinen Formel I.

In einer anderen Ausführungsform der Herstellung setzt man im Bereich von 0,00001 bis 0,001, besonders bevorzugt 0,0001 bis 0,0005 Äquivalente Manganverbindung ein, bezogen auf Dien der allgemeinen Formel I.

In einer Ausführungsform der Herstellung setzt man 1 bis 5 Äquivalente Ligand L der allgemeinen Formel II, bezogen auf Mangan, ein, bevorzugt 1,1 bis 2 Äquivalente.

Als Coliganden sind solche Verbindungen geeignet, die von Monocarbonsäuren, zwei- oder mehrwertigen Carbonsäuren oder Diaminen abgeleitet sind, d.h. Monocarbonsäuren, zwei- oder mehrwertigen Carbonsäuren und Diamine selbst sowie im Falle von Monocarbonsäuren, zwei- oder mehrwertigen Carbonsäuren insbesondere deren korrespondierende Alkalimetallsalze.

In einer Variante sind Coliganden von solchen Monocarbonsäuren bzw. zwei- oder mehrwertigen Carbonsäuren abgeleitet, deren pKₐ-Wert bzw. pKₐ¹-Wert in Wasser bei 25°C unter 7 liegt.

In einer Variante sind Coliganden abgeleitet von Oxalsäure (III.1), Dihydroxyfumarsäure (III.2), Weinsäure (III.3), Maleinsäure (III.4), Quadratsäure (III.5), 2-Sulfobenzoesäure (III.6) und N(p-Toluolsulfonyl)glycin (III.7):

HOOC-COOH III.1

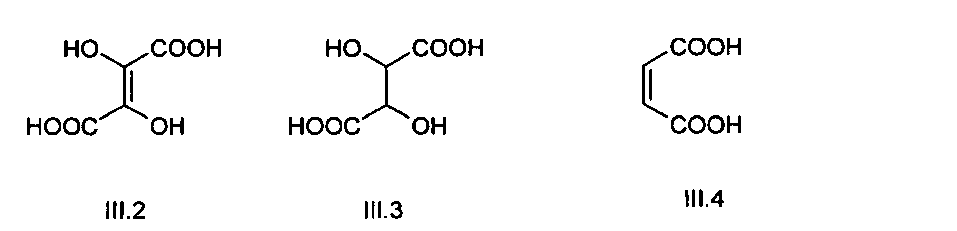

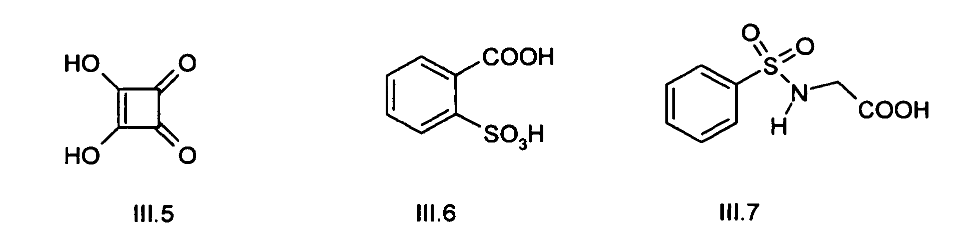

Weiterhin ist Ascorbinsäure geeignet.

Ein weiterer ganz besonders bevorzugter Coligand ist 1,2-Diaminocyclohexan, wobei sowohl das Isomerengemisch als auch die jeweiligen cis- und trans-Isomeren in angereicherter Form in Frage kommen.

In einer Variante setzt man Coliganden als Mischung von Monocarbonsäuren und Alkalimetallsalz der betreffenden Monocarbonsäure ein.

In einer Variante setzt man Coliganden als Mischung von zwei- oder mehrwertiger Carbonsäure und Alkalimetallsalz der betreffenden zwei- oder mehrwertigen Carbonsäure ein.

In einer Variante setzt man 0,1 bis 5 Äquivalente, bevorzugt 0,5 bis 1 Äquivalent Coligand ein, bezogen auf Mangan.

Man setzt Dien der allgemeinen Formel I mit mindestens einem Peroxid um, wobei man bis zu 4 Äquivalenten Peroxid pro Äquivalent C-C-Doppelbindung einsetzt. Vorzugsweise setzt man mindestens ein Äquivalent Peroxid pro Äquivalent C-C-Doppelbindung ein. Als Peroxid bevorzugt organische Peroxide einsetzen, insbesondere tert.-Butylhydroperoxid, Cumolhydroperoxid, 1,3-Diisopropylmonohydroperoxid, 1-Phenyl-ethylhydroperoxid. Wasserstoffperoxid (H₂O₂) ist als Peroxid besonders bevorzugt.

Wünscht man Wasserstoffperoxid einzusetzen, so setzt man es als wässrige Lösung ein, beispielsweise als 30 Gew.-% oder 50 Gew.-% Lösung, deren Gehalt an reaktionsfähigem H₂O₂ man nach sich bekannten Methoden, beispielsweise durch Titration, ermitteln kann.

In einer Variante setzt man bis zu 3, bevorzugt bis zu 2,1 Äquivalenten Peroxid pro Äquivalent C-C-Doppelbindung ein.

Für die Reihenfolge zur Kontaktierung der Reaktionspartner der Herstellung von Bisepoxid sind mehrere Vorgehensweisen möglich.

In einer Variante mischt man zunächst Ligand L der allgemeinen Formel II und Coligand mit Dien der allgemeinen Formel I und Peroxid und setzt anschließend Manganverbindung zu.

In einer anderen Variante mischt man zunächst Ligand L der allgemeinen Formel II mit Coligand und Dien der allgemeinen Formel I und Manganverbindung und setzt anschließend Peroxid zu.

In einer anderen Variante stellt man zunächst durch Kontaktieren von Manganverbindung und Ligand L und Coligand der allgemeinen Formel II eine Komplexverbindung her, die man anschließend mit Dien der allgemeinen Formel I und danach mit Peroxid mischt.

In einer anderen Variante stellt man zunächst durch Kontaktieren von Manganverbindung und Ligand L der allgemeinen Formel II eine Komplexverbindung her, die man anschließend mit Dien der allgemeinen Formel I und Coligand und danach mit Peroxid mischt.

In einer anderen Variante stellt man zunächst durch Kontaktieren von Manganverbindung und Ligand L der allgemeinen Formel II eine Komplexverbindung der Formel [LMn(µ-O)₃MnL]X her, die man anschließend mit Dien der allgemeinen Formel I und Coligand und danach mit Peroxid mischt.

In einer anderen Variante stellt man zunächst durch Kontaktieren von Manganverbindung und Ligand L und Coligand der allgemeinen Formel II eine Komplexverbindung her, die man anschließend mit Dien der allgemeinen Formel I und danach mit Peroxid mischt, wobei man Peroxid in zwei Portionen in einem Zeitabstand von mindestens 2 Stunden zugibt.

Es ist nicht genau bekannt, in welcher Form die katalytisch aktive Spezies vorliegt. Ohne einer Theorie den Vorzug geben zu wollen, erscheint es denkbar, dass Mangan während der katalytischen Reaktion zumindest zeitweise in der Oxidationsstufe +IV vorliegt. Weiterhin erscheint es möglich, dass während der katalytischen Reaktion zumindest zeitweise einfach oder mehrfach µ-Oxo-verbrückte Spezies vorliegen.

In einer Variante stellt man Bisepoxid in einem Lösungsmittel oder einem Gemisch von Lösungsmitteln her. Als Lösungsmittel kann man bei Reaktionstemperatur flüssige organische oder anorganische Flüssigkeiten verwenden, die unter den Bedingungen nicht oder nur in vernachlässigbaren Anteilen mit den Reaktionspartnern und Produkt, d.h. beispielsweise Bisepoxid, reagieren.

Geeignet sind beispielsweise C₁-C₄-Alkanole wie Methanol, Ethanol, n-Propanol, isoPropanol, weiterhin Ketone wie beispielsweise Aceton, Methylethylketon und Methylisobutylketon (MIBK), Acetonitril, halogenierte Kohlenwasserstoff wie beispielsweise Methylenchlorid, Chloroform, 1,1,2,2-Tetrachlorethan und Wasser. Besonders gut geeignet sind Gemische aus Wasser und Acetonitril, Gemische aus Wasser und Methanol und Gemische aus Wasser und Aceton.

In einer Variante arbeitet man mit so viel Lösungs-mittel bzw. Gemisch von Lösungsmitteln, dass die Konzentration von Bisepoxid 50 Gew.-% nicht überschreitet, bevorzugt 5 bis 15 Gew.-%.

In einer Variante stellt man Bisepoxid her, ohne den Katalysator vorher auf einem oder mehreren festen Trägermaterialien wie beispielsweise Kieselgel oder Aluminiumoxid immobilisiert zu haben.

In einer Variante stellt man Bisepoxid bei Temperaturen im Bereich von -50 bis 100°C, bevorzugt von -30 bis 80°C, besonders bevorzugt von -10 bis 60°C und ganz besonders bevorzugt von 0 bis 5°C her.

In einer Variante stellt man Bisepoxid bei einem Druck im Bereich von 1 bis 200 bar, bevorzugt bei 1 bis 100 bar, besonders bevorzugt bei Normaldruck bis 10 bar durch.

In einer Variante stellt man Bisepoxid bei einem pH-Wert von 1 bis 7, bevorzugt von 3 bis 5 her.

In einer Variante beträgt die Reaktionsdauer 1 Minute bis 24 Stunden, bevorzugt 30 Minuten bis 20 Stunden.

Als Reaktionsgefäße für die Herstellung von Bisepoxid sind im Prinzip alle gängigen Reaktionsgefäße geeignet, beispielsweise Rohrreaktoren und Rührkessel, wobei man Rührkessel absatzweise oder kontinuierlich betreiben kann und Rohrreaktoren vorzugsweise kontinuierlich.

In Schritt (a) erhält man Lösungen von Bisepoxid. Die so erhältlichen Lösungen von Bisepoxid können geringe Anteile an Monoepoxid, beispielsweise der Formel V.1 oder V.2, 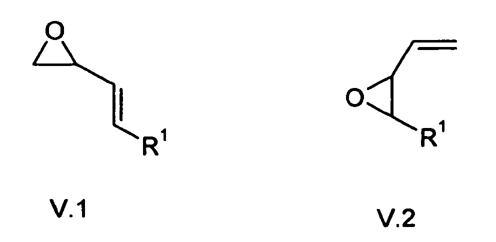 enthalten, wobei der Anteil an Monoepoxid in der Regel unter 8 mol-%, bezogen auf reines Bisepoxid, beträgt. Man kann Bisepoxid aus den Lösungen isolieren und aufreinigen.

In Schritt (b) setzt man ohne Isolierung von in Stufe (a) hergestelltem Bisepoxid in Gegenwart mindestens eines basischen Katalysators mit H₂S um.

In einer Ausführungsform setzt man Lösungen von Bisep-oxid ein, welche nach einem vorstehend beschriebnen Verfahren erhältlich sind, und verzichtet auf Isolierungs- und Aufreinigungsoperationen.

In einer Ausführungsform setzt man in Stufe (b) mit 1 bis 10 Äquivalenten H₂S um, bevorzugt mit 1 bis 2 Äquivalenten H₂S, bezogen auf Äquivalent Epoxidgruppe.

Stufe (b) des Herstellungsverfahrens übt man in Gegenwart mindestens eines basischen Katalysators aus.

Als basische Katalysatoren sind basische Alkalimetallsalze und Ammoniumsalze geeignet, beispielsweise Alkalimetallhydroxide, Alkalimetallcarbonate, Alkalimetallhydrogensulfide, Ammoniumhydroxide. Als Alkalimetallkationen sind beispielsweise Li⁺, Na⁺, K⁺, Rb⁺ und Cs⁺, insbesondere Na⁺ und K⁺ zu nennen.

Als Ammoniumionen sind nicht nur unsubstituiertes NH₄⁺, zu nennen, sondern auch ein- und bis zu vierfach alkyliertes Ammonium zu nennen, beispielsweise N(R⁴)(R⁵)(R⁶)(R⁷)⁺, wobei R⁴ bis R⁷ jeweils gleich oder verschieden sind und ausgewählt aus Wasserstoff, Benzyl, C₁-C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl oder n-Decyl, besonders bevorzugt C₁-C₄-Alkyl wie Methyl, Ethyl, n-Propyl, iso-Propyl, n-Butyl, iso-Butyl, sec.-Butyl und tert.-Butyl, Phenyl oder CH₂-CH₂-OH. Beispielhaft seien Tetramethylammonium, Tetraethylammonium, Benzyltrimethylammonium, Methyldiethanolammonium und n-Butyldiethanolammonium genannt.

Bevorzugt ist mindestens ein basischer Katalysator in Stufe (b) gewählt aus Alkalimetallhydrogensulfid, Alkalimetallhydroxid und Benzyltri(C₁-C₁₀-Alkyl)ammoniumhydroxid, ganz besonders bevorzugt sind Natriumhydrogensulfid, Kaliumhydrogensulfid, Natriumhydroxid, Kaliumhydroxid und Benzyltrimethylammoniumhydroxid.

In einer Ausführungsform von Schritt (b) setzt man im Bereich von 10⁻⁴ bis 10 Gew.-%, bevorzugt 0,5 bis 5 Gew.-% basischen Katalysator ein, bezogen auf Bisepoxid.

In einer Ausführungsform der vorliegenden Erfindung führt man Stufe (b) bei einem Druck im Bereich von 1 bis 200 bar, bevorzugt bei 1 bis 100 bar, besonders bevorzugt bei 1 bis 10 bar aus.

In einer Ausführungsform führt man Stufe (b) bei einer Temperatur im Bereich von - 50 bis 100 °C, bevorzugt von -30 bis 80 °C, besonders bevorzugt von -10 bis 60°C, ganz besonders bevorzugt von 15 bis 35 °C aus.

In einer Ausführungsform führt man Stufe (b) bei einem pH-Wert von 8 bis 13, bevorzugt von 9 bis 11 durch.

In einer Variante geht man von einer nach Stufe (a) des erfindungsgemäßen Verfahrens erhältlichen Lösung von Bisepoxid aus, gibt H₂S zu, gibt danach mindestens einen basischen Katalysator zu und lässt reagieren.

In einer Variante kann man in Stufe (b) des erfindungsgemäßen Zweistufenverfahrens weiteres Lösungsmittel, gewählt aus den oben unter Stufe (a) aufgeführten Lösungsmitteln, zugeben.

In einer Variante beträgt die Reaktionsdauer von Stufe (b) 10 min bis 4 h, besonders bevorzugt 0,5 Stunden bis 2 Stunden.

Als Reaktionsgefäße für die Ausübung des erfindungsgemäßen Zweistufenverfahrens sind im Prinzip alle gängigen Reaktionsgefäße geeignet, beispielsweise Rohrreaktoren und Rührkessel, wobei man Rührkessel absatzweise oder kontinuierlich betreiben kann und Rohrreaktoren vorzugsweise kontinuierlich. Auch kontinuierlich betriebene Rührkesselkaskaden sind als geeignete Gefäße denkbar.

Ohne einer Theorie den Vorzug geben zu wollen, erscheint es denkbar, dass durch gegebenenfalls überschüssiges H₂S in Stufe (b) nicht abreagiertes Peroxid aus Stufe (a) abgefangen wird.

Durch Ausübung des erfindungsgemäßen Herstellungsverfahrens erhält man Lösungen von Dithiolgemischen bzw. korrespondierenden Salzen von Dithiolen, die ebenfalls ein Gegenstand der vorliegenden Erfindung sind. Aus erfindungsgemäßen Lösungen von Dithiolgemischen lassen sich Dithiolgemische bzw. ihre korrespondierenden Salze nach an sich bekannten Methoden isolieren, beispielsweise Neutralisieren, Abdestillieren des oder der Lösungsmittel. Um besonders reines Dithiolgemisch zu erhalten, kann man destillieren, beispielsweise unter vermindertem Druck.

Durch an sich bekannte Methoden wie beispielsweise Chromatographie kann man erfindungsgemäße Dithiolgemische auftrennen in erythro- und threo-Dithiol, und durch chirale Diskriminierung kann man die Enantiomeren von threo-Dithiol trennen oder anreichern.

Die Erfindung wird durch Arbeitsbeispiele erläutert.
1. Herstellung eines Gemischs aus 40 mol-% erythro-1,4-Dimercaptobutan-2,3-diol und 60 mol-% racemischem threo-1,4-Dimercaptobutan-2,3-diol
   (a) Herstellung von Bisepoxid
      (a.1)
         In einen 150-ml-Glasautoklaven mit Einlassrohr wurden miteinander vermischt:
         42,6 g Acetonitril,
         9 ml wässrige Mangan(II)-acetat-Lösung mit einer Konzentration von 0,02 mol Mn/l
         9 ml 1,4,7-Trimethyl-1,4,7-triazacyclononan mit einer Konzentration von 0,03 mol/l
         9 ml wässriger Natriumoxalat/Oxalsäurepuffer (Molverhältnis: 1:1) mit einer Konzentration von 0,06 mol/l aus Summe von Oxalat und Oxalsäure.

Die so erhältliche Lösung wurde mit Hilfe eines Trockeneis/Aceton-Bades auf etwa -40°C gekühlt. Anschließend wurden 3,34 g (61,8 mmol) 1,3-Butadien einkondensiert. Anschließend wurde mit Hilfe eines Eisbads eine Temperatur von 0°C eingestellt.

Anschließend wurden innerhalb von 1 Stunde 16,7 g 50 Gew.-% wässrige H₂O₂-Lösung (246 mmol H₂O₂) zugepumpt, wobei darauf geachtet wurde, dass die Temperatur nicht über 25°C stieg. Man beobachtete, dass der Druck im Autoklaven auf 5,2 bar stieg. Danach wurde das Eisbad entfernt und 2 Stunden bei Zimmertemperatur nachgerührt. Danach hatte sich ein Druck von 3,2 bar eingestellt.

Anschließend wurden 16,6 g 50 Gew.-% wässrige H₂O₂-Lösung (244 mmol H₂O₂) zugepumpt, wobei darauf geachtet wurde, dass die Temperatur nicht über 25°C stieg.

Man beobachtete, dass der Druck im Autoklaven auf 3,8 bar stieg. Danach wurde das Eisbad entfernt und 5,5 Stunden bei Zimmertemperatur gerührt. Nach 5,5 Stunden hatte sich ein Druck von 5 bar eingestellt.

Anschließend wurde entspannt und die Zusammensetzung der resultierenden hellen Lösung (94,6 g) durch Gaschromatographie ermittelt. Man ermittelte einen Gehalt von 58,4 mmol Bisepoxid der Formel VII.1 und 3,4 mmol Vinyloxiran IV.1.1

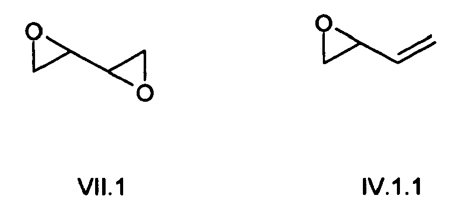

Die Ausbeute an gewünschtem Bisepoxid VII.1 betrug 95,5 %.
(a.2)
   Die Reaktion aus (a.1) wurde wiederholt, jedoch wurde nach dem Zupumpen von 16,7 g 50 Gew.-% wässriger H₂O₂-Lösung und Entfernung des Eisbads 12 Stunden bei Zimmertemperatur gerührt. Anschließend wurde wie unter (a.1) beschrieben weitergearbeitet. Man erhielt Bisepoxid VII.1 in sehr guter Ausbeute.
(b) Herstellung von erfindungsgemäßem Dithiolgemisch
   50 g der aus 1 (a.1) resultierenden Lösung wurden in einem 400-ml-Glasautoklaven vorgelegt und bei Zimmertemperatur mit 6 bar H₂S aufgepresst. Danach wurde eine Lösung von 1,04 g NaOH (fest) in 20 ml Methanol mit Hilfe einer HPLC-Pumpe zugesetzt. Während der Zugabe von Methanol/NaOH wurde eine Temperaturerhöhung von 25 auf 35 °C beobachtet.

Dabei wurde durch kontinuierliches Aufpressen von H₂S der Druck auf 6 bar gehalten. Danach wurden die Leitungen der HPLC-Pumpe mit 50 ml Acetonitril nachgespült.

Nach beendeter Reaktion, was sich durch Abklingen der Temperatur bemerkbar machte, wurde der Autoklav entspannt und über einen Zeitraum von 14 Stunden durch Durchleiten von N₂ durch die Reaktionsmischung von überschüssigem H₂S befreit.

Man erhielt 83,8 g einer klaren Lösung von Dithiolgemisch. Das erhaltene erfindungsgemäße Dithiolgemisch IV.1 wurde gaschromatographisch getrennt und hatte gemäß Gaschromatographie die folgende Zusammensetzung:
40 mol-% erythro-IV.1
60 mol-% threo-IV.1 als Racemat.

Bedingungen für die Gaschromatographie: Säule: HP-5, Länge: 30 m, Innendurchmesser= 0,25 mm, Filmdicke 0,25 µm, Detektor: WLD, Init. T.: 40°C, Init. Zeit: 5 min, Rate: 10°C/min, Finale Temperatur 290°C, Retentionszeit IV.1: 18,00-18,50 min.

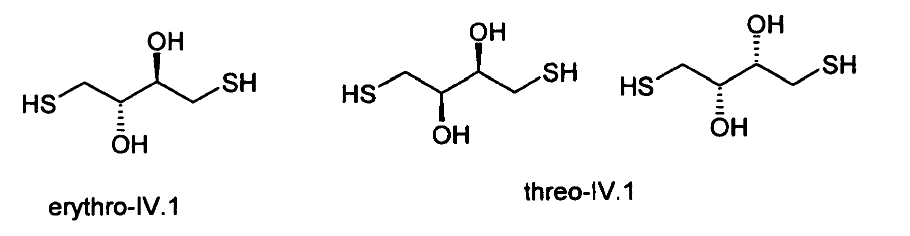

### 2. Behandlung von Blößen mit erfindungsgemäßem Dithiolgemisch

Die Werte in Gew.-% beziehen sich jeweils auf das Salzgewicht, wenn nicht anderes angegeben ist.

### Allgemeine Vorbehandlung:

Die Haut eines Süddeutschen Rindes wurde zunächst bei 28°C mit 200 Gew.-% Wasser und 0,2 Gew.-% C₁₅H₃₁-O-(CH₂-CH₂-O)₇-H 10 Minuten in einem Fass bei leichtem Umrühren vorgeweicht. Die Flotte wurde abgelassen und danach mit 100 Gew.-% Wasser, 0,2 Gew.-% C₁₅H₃₁-O-(CH₂-CH₂-O)₇-H und 0,5 Gew.-% Na₂CO₃ bei gelegentlichem Rühren 19 Stunden eingeweicht. Anschließend wurde die Flotte abgelassen.

Die geweichten Häute süddeutscher Rinder wurden grün entfleischt (Stärke etwa 4 mm) und die Croupons der Häute in Hautstücke zu je 2,5 kg Grüngewicht geschnitten.

Im Folgenden beziehen sich die Werte in Gew.-% jeweils auf das Grüngewicht, wenn nicht anderes vermerkt.

### 2.1. Äscher des Vergleichsbeispiels V1

Für das Vergleichsbeispiel V1 wurden 100 Gew.-% Grüngewicht in einem drehbaren 10-I-Fass mit strömungsbrechenden Inneneinbauten nacheinander mit 60 Gewichtsteilen Wasser, 0,8 Gew.-% NaSH und 3 Gew.-% Kalkhydrat beaufschlagt. Es folgten im Abstand von 30 Minuten je 0,75 Gew.-% Natriumsulfid. Das Fass wurde weitere 45 Minuten bei 15 Umdrehungen/Minute betrieben. Anschließend wurden weitere 40 Gewichtsteile Wasser dosiert. Nach 10 Stunden bei 23 bis 27°C und 5 Umdrehungen/Minute wurden die Versuche beendet, indem die Flotte abgelassen wurde und die Haute zweimal 15 Minuten mit 150 Gewichtsteilen Wasser gewaschen wurden.

### 2.2. Haarzerstörender Äscher der erfindungsgemäßen Beispiele 2.1 bis 2.4

In den erfindungsgemäßen Beispielen 2.1 bis 2.4 wurden 100 Gew.-% Grüngewicht in drehbare 10-I-Fässer mit strömungsbrechenden Inneneinbauten zunächst mit 60 Gew.-% Wasser versetzt und anschließend wie aus Tabelle 1 ersichtlich mit Produkten beaufschlagt.

**Tabelle 1**

| Beispiel | Einsatzmenge [Gew.-%] | Produkt | Zeit [min] |
|---|---|---|---|
| 2.1 | 0,5 | Natriumsulfhydrat (70%) | |
| | 0,5 | Dithiolgemisch IV.1 | 60 |
| | 1,2 | Kalkhydrat | 60 |
| | 1,2 | Kalkhydrat | 60 |
| 2.2 | 1,0 | Dithiolgemisch IV.1 | 60 |
| | 1,2 | Kalkhydrat | 60 |
| | 1,2 | Kalkhydrat | |
| 2.3 | 1,5 | Dithiolgemisch IV.1 | 60 |
| | 1,2 | Kalkhydrat | 60 |
| | 1,2 | Kalkhydrat | 60 |
| 2.4 | 1,0 | Dithiolgemisch IV.1 | 60 |
| | 1,0 | Wässrige Natriumhydroxid-Lsg. (50 Gew.-%) | 30 |
| | 1,0 | Wässrige Natriumhydroxid-Lsg. (50 Gew.-%) | 30 |
| | 50 | Wasser | |
| | 0,4 | Wässrige Natriumhydroxid-Lsg. (50 Gew.-%) | 60 |
| | 50 | Wasser | 30 |

Die Fässer wurden weitere 45 Minuten bei 5 Umdrehungen/Minute betrieben. Anschließend wurden weitere 40 Gew.-% Wasser dosiert. Nach 10 Stunden bei 23 bis 27°C bei periodischem Betreiben mit 3 Umdrehungen/Minute über jeweils 5 Minuten pro Stunde wurden die Versuche beendet, indem die Flotten abgelassen wurden und die erhaltenen Blößen zweimal für je 15 Minuten mit 150 Gew.-% Wasser gewaschen wurden.

### 2.3. Beurteilung von Blößen gemäß Vergleichbeispiel und gemäß erfindungsgemäßer Beispiele nach dem Äscher

Die gemäß den erfindungsgemäßen Beispielen behandelten Blößen waren den nach Vergleichsbeispiel V1 behandelten Häuten hinsichtlich der Schwellung nur wenig überlegen, zeichneten sich aber durch einen glatteren und flacheren Narben aus, insbesondere die Blößen der erfindungsgemäßen Beispiele 2.3 bis 2.4. Die Epidermis und die Haare mit Haarwurzel in den Blößen nach Beispiel 2.1 bis 2.3 waren weitgehend und in Blöße nach Beispiel 2.4 vollständig zerstört.

## Patentansprüche

1. Dithiolgemische, enthaltend im Bereich von
(A) 55 bis 65 mol-% erythro -Dithiol und
(B) 35 bis 45 mol-% threo-Dithiol der allgemeinen Formel IV 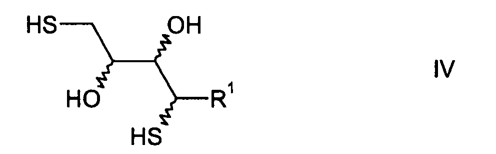 und ihre korrespondierenden Alkalimetallsalze und Ammoniumsalze,
wobei R¹ gewählt wird aus Wasserstoff und C₁-C₁₂-Alkyl, unsubstituiert oder substituiert mit einer oder mehreren S-H oder O-H-Gruppen.

2. Dithiolgemische nach Anspruch 1, **dadurch gekennzeichnet, dass** (B) als Racemat vorliegt.

3. Wässrige Lösungen, enthaltend Dithiolgemische nach Anspruch 1 oder 2.

4. Verwendung von Dithiolgemischen nach Anspruch 1 oder 2 oder wässriger Lösungen nach Anspruch 3 zum Behandeln von Häuten toter Tiere.

5. Verfahren zum Entfernen von Hornsubstanzen aus Häuten toter Tiere unter Verwendung von mindestens einem Dithiolgemisch nach Anspruch 1 oder 2 oder mindestens einer wässrigen Lösung nach Anspruch 3.

## Claims

1. A dithiol mixture comprising
(A) from 55 to 65 mol% of erythro-dithiol and
(B) from 35 to 45 mol% of threo-dithiol of the formula IV 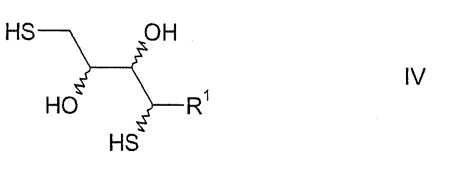 and their corresponding alkali metal salts and ammonium salts,
where R¹ is selected from hydrogen and C₁-C₁₂-alkyl, unsubstituted or substituted by one or more S-H or O-H groups.

2. The dithiol mixture according to claim 1, wherein (B) is present as a racemate.

3. An aqueous solution comprising a dithiol mixture according to claim 1 or 2.

4. The use of a dithiol mixture according to claim 1 or 2 or an aqueous solution according to claim 3 for treating hides of dead animals.

5. A process for removing horny substances from hides of dead animals using at least one dithiol mixture according to claim 1 or 2 or at least one aqueous solution according to claim 3.

## Revendications

1. Mélanges de dithiols, contenant, dans une plage de
(A) 55 à 65% en mole, de l'érythro-dithiol et
(B) 35 à 45% en mole, du thréo-dithiol de formule générale IV 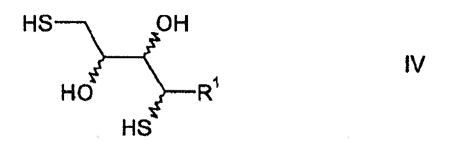 et leurs sels de métal alcalin et sels d'ammonium correspondants,
où R¹ est choisi parmi hydrogène et C₁-C₁₂-alkyle, non substitué ou substitué par un ou plusieurs groupes S-H ou 0-H.

2. Mélanges de dithiols selon la revendication 1, **caractérisés en ce que** (B) se trouve sous forme de racémate.

3. Solutions aqueuses, contenant des mélanges de dithiols selon la revendication 1 ou 2.

4. Utilisation de mélanges de dithiols selon la revendication 1 ou 2 ou de solutions aqueuses selon la revendication 3 pour le traitement de peaux d'animaux morts.

5. Procédé pour enlever des substances cornées de peaux d'animaux morts avec utilisation d'au moins un mélange de dithiols selon la revendication 1 ou 2 ou d'au moins une solution aqueuse selon la revendication 3.
